## Europäisches Patentamt

## European Patent Office

## Office européen des brevets

(11) Publication number: **0 076 159**
**B1**

(12)

# EUROPEAN PATENT SPECIFICATION

(45) Date of publication of patent specification: **27.08.86**

(51) Int. Cl.⁴: **A 61 K 7/06**

(21) Application number: **82305156.0**

(22) Date of filing: **29.09.82**

(54) Hair growing agent or hair tonic.

(30) Priority: **30.09.81 JP 155492/81**
**29.07.82 JP 132507/82**

(43) Date of publication of application:
**06.04.83 Bulletin 83/14**

(45) Publication of the grant of the patent:
**27.08.86 Bulletin 86/35**

(84) Designated Contracting States:
**BE DE FR GB IT NL SE**

(56) References cited:
**FR-A-1 159 138**
**GB-A-2 032 974**

(73) Proprietor: **MEDICAL HAIR RESEARCH KABUSHIKI KAISHA**
**3-31-9, Asagaya-Minami**
**Suginami-ku Tokyo 106 (JP)**

(73) Proprietor: **Inaba, Masumi**
**3-31-9, Asagaya-Minami**
**Suginami-ku Tokyo 106 (JP)**

(72) Inventor: **Inaba, Masumi**
**3-31-9, Asagaya-Minami Suginami-ku**
**Tokyo 106 (JP)**

(74) Representative: **Spencer, Graham Easdale et al**
**A.A. Thornton & CO Northumberland House**
**303-306, High Holborn**
**London WC1V 7LE (GB)**

Courier Press, Leamington Spa, England.

**Description**

The present invention is concerned with a method of developing and growing terminal hairs from the fine (vellus) hairs on a hairless or bald scalp area.

Male pattern baldness is a condition in which coarse hairs become thinner and shorter each time they regenerate until eventually they become vellus hairs; however hair follicles do not stop functioning.

Various theories have been proposed regarding the causes of male pattern baldness. These include, for example, the seborrhoea theory that an increased secretion of sebum from the sebaceous glands mixes with the dirt of the scalp to form seborrhoea which then clogs the hair follicle and causes a nutritional or circulatory disorder of the hair root, the hormone theory that excessive production of the male sex hormone testosterone is responsible by causing an imbalance of the hormone in the scalp, and other theories associated with genetics and with nutrition.

We believe that these theories are based on an incomplete understanding of the trichology of hair development.

For a better understanding of the invention and the prior art, reference will be made in the following description to the accompanying drawings, in which:

Figure 1 is a cross-section of the scalp showing the various stages of the hair growth cycle, in which A is the anagen stage, B is the catagen stage, C is the telogen stage, and D is the growth period consisting of (a) the isthmal (hair germ) stage, (b) the peg stage, (c) the bulbous peg stage, and (d) the growth of the terminal hair; and

Figure 2 is a cross-section of a hair follicle indicating the paths by which the male sex hormone testosterone acts on the development of the hair.

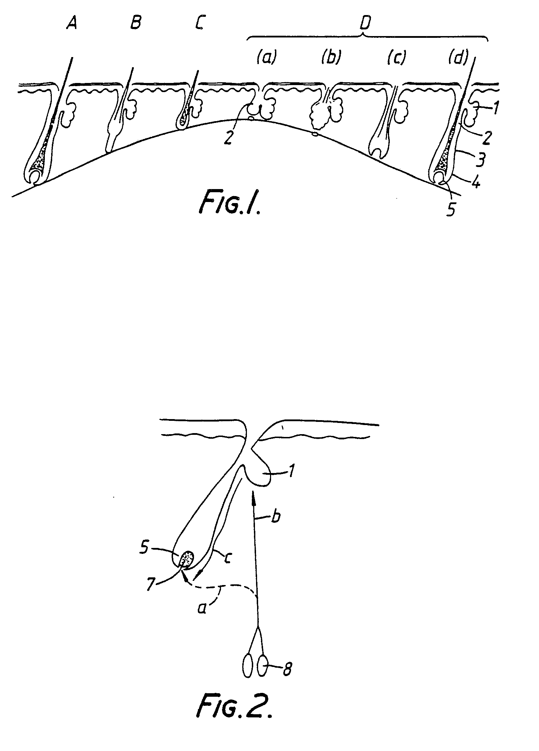

The hair growth cycle consists of an anagen stage (the development phase), a catagen stage (the intermediate phase), and a telogen stage (the pause phase). As indicated in Figure 1, the duct of the sebaceous gland (1) opens into the isthmal portion (2) of the hair follicle (3). During the anagen stage, a hair develops from the aggregation of matrix cells (5) in the hair bulb (4). However, the hair does not continue to grow indefinitely. At the end of the catagen stage, after an intermediate period, the hair falls out and regeneration of a new hair begins (telogen stage).

It is commonly believed that the regeneration of hair begins only in the lower portion of the telogen hair follicle. A hair bud is formed and moves downward from the base of the telogen hair follicle. When the hair bulb is formed, the matrix cells within the hair bulb, which occupy an invagination of the dermal papilla, differentiate and keratinise to form a new hair.

On the basis of this conventional theory, previously known hair-growing compositions commonly have as their principal purpose the promotion of good blood circulation in the hair bulb. They contain as a major ingredient, for example, hinokiol, menthol, or capsicum tincture. Such ingredients may promote favourable blood circulation in the scalp and secondarily improve the blood circulation of the hair bulb, with or without consequent growth or development of the hair. Such conventional hair-growing compositions, however, have generally proved unsatisfactory in promoting hair growth.

We have now developed a hair-growing composition which is based upon a new theory of hair development, not the conventional theory as described above.

In order that the invention may be more fully understood, the new theory upon which the invention is based will be described with reference to Figure 1.

According to the conventional theory described above, hair begins to regenerate in the lower portion of the telogen hair follicle. According to the new theory upon which the present invention is based, a hair bud can also develop from the isthmal portion of the trichilemma (outer root sheath) (2), close to the duct opening of the sebaceous gland, during the so-called hair germ stage of the growth period; the new hair then begins to form in the epithelial germ cells located in the lower part of the hair follicle. The hair follicle continues to descend while forming the new hair (peg stage) and the hair bulb (4) is formed. The follicle continues to descend until the hair matrix (5) is formed within the hair bulb (bulbous peg stage). When the matrix begins to function effectively, the follicle stops descending and the new (terminal) hair grows to the skin surface. Hairs, then, are formed not only from within the matrix of the hair bulb, as commonly believed, but can also be formed from the epithelial cells in the lower part of the hair follicle prior to formation of the hair bulb. This process is quite similar to the keratinisation of the epithelium. The sole difference is that the process of keratinisation within the hair follicle requires the active presence of the sebaceous gland. According to the new theory, known as the sebaceous gland or Inaba theory, the sebaceous gland plays an important role in hair development.

In order to understand the mode of action of the compositions of the present invention, the chemical control of hair development will be briefly described with reference to Figure 2.

Testosterone, the male sex hormone formed in the testicles (8), is known to be involved in hair development. It has been reported that it acts directly on the matrix cells (5) through the hair papilla (7) of the hair bulb, as indicated by the dotted line *a* in Figure 2.

However, it has now been found that testosterone is carried directly by the blood to the sebaceous gland (1), as well as to the hair bulb, as indicated by the solid line *b* in Figure 2. It has also been found that the testosterone which is carried to the sebaceous gland is then reduced by the 5 - alpha - reductase enzyme present in the gland to 5 - alpha - dehydrotestosterone (hereinafter referred to as 5-alpha-DHT). The 5 - alpha - DHT, a more powerful hormone than testosterone, is then carried via the blood vessels surrounding the hair follicle to the hair papilla (7) and the matrix cells (5), as indicated by the solid line c in Figure 2, where it reduces or suppresses the mitotic activity of the matrix cells (5), thereby inhibiting hair development.

The sebaceous gland may become so enlarged by the effects of nutrition or hormone activity that the amount of 5 - alpha - reductase in the gland is significantly increased; on reaction with testosterone it is converted to 5 - alpha - DHT which gradually reduces the mitotic activity of the hair follicles, causing the terminal hairs to revert to vellus hairs and leading to a condition of male pattern baldness. Although testosterone or a small amount of 5 - alpha - DHT accelerates hair development, the presence of a large amount of 5 - alpha - DHT suppresses the mitotic activity of the matrix cells.

It has now been found that the amount of testosterone which is converted by 5 - alpha - reductase into 5 - alpha - DHT can be reduced by reducing or suppressing the enzymatic activity of the 5 - alpha - reductase present in the sebaceous glands, thereby causing a reduction or elimination of the suppressive action of 5 - alpha - DHT on the matrix cells.

Attempts have been reported in which a competitive antagonist such as progesterone, lutein, or a male sex hormone has been used to regulate the enzymatic activity of 5 - alpha - reductase. These antagonists, however, are not suitable additives for use in hair-growing compositions because of their undesirable side effects.

In accordance with the new theory described above, we have developed a hair-growing composition which comprises a physiologically acceptable strong oxidising agent capable of reducing or suppressing the enzymatic activity of 5-alpha-reductase in the sebaceous glands.

According to the present invention, therefore, there is provided a method for the cosmetic treatment of male pattern baldness, which comprises repeatedly applying a composition containing a physiologically acceptable strong oxidising agent to the bald scalp so as to bring the composition into contact with the sebacous glands of the scalp.

Suitably substances for use in the compositions of the invention include, for example, stabilised chlorine dioxide, potassium bromate, sodium perborate and sodium bromate. Any physiologically acceptable substance having the necessary oxidising strength can be used. The chlorine dioxide is chemically stabilised by any of the methods disclosed in U.S. Patents 4296103, 3147124, 3123521, and 2701781, such as the addition of sodium hydroxide. Stabilised chlorine dioxide is non-toxic to the human body at various concentrations and in compositions containing it as an additive.

The amount of oxidising agent used in the hair-growing compositions of the present invention varies according to the oxidising agent used. Thus stabilised chlorine dioxide is preferably used in amounts of from 0,01% to 0,05% (100 to 500 ppm), sodium bromate in amounts of from 5% to 12% (50,000 to 120,000 ppm), potassium bromate at concentrations of from 5% to 12%, and sodium perborate at concentrations of about 5%.

The oxidising agents are used in the compositions of the invention at concentrations which are sufficient to reduce or suppress the enzymatic activity of 5 - alpha - reductase without causing any undesirable effects on the human body. The compositions may be applied, together with other ingredients if desired, several times per day to an area of bald scalp. The hair-growing composition can be repeatedly applied over a long period of time. After application of the composition, the treated scalp area is preferably massaged; the oxidising agent present in the composition penetrates via the hair pouches of the scalp into the sebaceous glands and the enzymatic activities of the 5 - alpha - reductase and the reductive coenzymes (NADH, NADPH, and TPNH (triphosphopyridine nucleotide)) in glands are reduced or suppressed. As a result, only a small amount of the testosterone carried to the sebacous glands from the testicles is converted to 5-alpha-DHT, so that only unconverted testosterone and a small amount of 5-alpha-DHT are available to act on the matrix cells. Although it has been reported that 5 - alpha - reductase can be present in the sebaceous glands in large amounts and in the hair bulbs in smaller amounts, the compositions of the present invention are still effective because only a small amount of 5-alpha-DHT is formed from the testosterone carried directly to the matrix cells from the testicles, thereby promoting hair development.

It is noted that the testosterone or a small amount of 5-alpha-DHT can also have a favourable effect on the development of hairs on the chest after male puberty. As shown in Figure 1, the hair follicles form and grow to produce active segmentation of the matrix cells, from which the terminal hairs develop.

In order that the invention may be more fully understood, the following examples are given by way of illustration only.

Examples

Clinical tests were carried out on compositions comprising chlorine dioxide, sodium bromate, sodium perborate, and potassium bromate. Each test was performed on 5 males ranging in age from 30 to 50. The test composition was applied three times within a 5-hour period. The chlorine dioxide used was a stabilised aqueous solution comprising about 25% of sodium chloride and 4—5% of sodium hydroxide as a stabiliser, and having a chlorine dioxide concentration of 5—

15%; this aqueous solution was diluted to give the desired concentrations.

The results of the tests are shown in the following Table. The effects reported are average results based on the individual data obtained from each test.

TABLE

| Oxidizing agent | Concentration | Effect after application | | |
|---|---|---|---|---|
| | | After 1 month | After 3 months | After 6 months |
| Stabilized chlorine dioxide aqueous solution | 50 ppm | Vellus slightly elongated | Vellus slightly elongated | Vellus slightly elongated |
| Stabilized chlorine dioxide aqueous solution | 100 ppm | Vellus slightly elongated; terminal hairs developed epilation decreased | Development of terminal hairs accelerated; some elongation of vellus | Development of terminal hairs accelerated; their elongation slight |
| Stabilized chlorine dioxide aqueous solution | 250 ppm | Vellus slightly elongated terminal hairs developed; epilation decreased | About 5 mm terminal hairs grown | About 15 mm terminal hairs grown |
| Stabilized chlorine dioxide aqueous solution | 500 ppm | Vellus slightly elongated terminal hairs developed; epilation decreased | About 8 mm terminal hairs grown | About 20 mm terminal hairs grown grown with some brown color |
| Sodium bromate solution | 5% | Vellus slightly elongated | Vellus slightly elongated | Vellus slightly elongated |
| Sodium bromate solution | 8% | Vellus slightly elongated | Terminal hairs grown | Terminal hairs elongated |
| Sodium bromate solution | 12% | Vellus slightly elongated | Terminal hairs grown | Terminal hairs elongated |
| Sodium perborate solution | 5% | Vellus slightly elongated | Vellus slightly elongated | Vellus slightly elongated |
| Sodium perborate solution | 8% | Vellus slightly elongated | Terminal hairs grown with brown color | Turning considerably brown (inappropriate for use) |
| Sodium perborate solution | 12% | Vellus slightly elongated | Terminal hairs grown with brown color | Turning considerably brown (inappropriate for use) |
| Poatassium bromate solution | 5% | Vellus slightly elongated | Vellus slightly elongated | Vellus slightly elongated |
| Potassium bromate solution | 8% | Vellus slightly elongated | Terminal hairs grown and slightly elongated | Terminal hairs grown; elongation; proceeds |
| Potassium bromate solution | 12% | Vellus slightly elongated | Terminal hairs grown and slightly elongated | Terminal hairs grown; slight elongation |

It will be seen that the results shown in the Table appear to support the new theory of hair growth on which the compositions of the present invention are based.

In contrast to conventional hair-growing compositions which are intended to merely improve deficiencies associated with nutritional and circulatory disorders of the hair bulb, the compositions of the present invention are intended to reduce or suppress the enzymatic activity of 5-alpha-reductase in the sebacous glands of the scalp in order to prevent its conversion into 5-alpha-DHT, which as indicated has a strong suppressing action on hair growth, and thereby eliminate the factor which controls the segmentation of the matrix cells; as a result, mainly unconverted testosterone acts on the matrix cells, which accelerates the mitotic division of the cells and turns the vellus hairs on the scalp into terminal hairs. These effects are not observed with conventional hair-growing compositions.

## Claims

1. A method for the cosmetic treatment of male pattern baldness, which comprises repeatedly applying a composition containing a physiologically acceptable strong oxidising agent to the bald scalp so as to bring the composition into contact with the sebacous glands of the scalp.

2. A method according to claim 1, in which the oxidising agent is stabilised chlorine dioxide, sodium bromate, potassium bromate, or sodium perborate.

3. A method according to claim 2, in which stabilised chlorine dioxide is present in the composition in an amount of from 0,01% to 0,05% (100 to 500 ppm).

4. A method according to claim 2, in which sodium bromate is present in the composition in an amount of from 5% to 12% (50,000 to 120,000 ppm).

5. A method according to claim 2, in which the potassium bromate is present in the composition in an amount of from 5% to 12%.

6. A method according to claim 2, in which sodium perborate is present in the composition in an amount of about 5%.

## Patentansprüche

1. Verfahren zur kosmetischen Behandlung des männlichen Typs der Glatzenbildung, welche die wiederholte Anwendung einer Zusammensetzung eines physiologisch verträglichen stark oxydierenden Wirkstoffes auf die kahle Kopfhaut umfaßt, um die Zusammensetzung in Kontakt mit den Talgdrüsen der Kopfhaut zu bringen.

2. Verfahren nach Anspruch 1, bei welchem der oxydierende Wirkstoof stabilisiertes Chlordioxyd, Natriumbromat, Kaliumbromat oder Natriumperborat ist.

3. Verfahren nach Anspruch 2, bei welchem stabilisiertes Chlordioxyd in einem Anteil von 0,01% bis 0,05% (100 bis 500 ppm) in der Zusammensetzung vorliegt.

4. Verfahren nach Anspruch 2, bei welchem Natriumbromat in einem Anteil von 5% bis 12% (50 000 bis 120 000 ppm) in der Zusammensetzung vorliegt.

5. Verfahren nach Anspruch 2, bei welchem Kaliumbromat in einem Anteil von 5% bis 12% in der Zusammensetzung vorliegt.

6. Verfahren nach Anspruch 2, bei welchem Natriumperborat in einem Anteil von ungefähr 5% in der Zusammensetzung vorliegt.

## Revendications

1. Un procédé pour le traitement cosmétique de la calvitie mâle, procédé qui consiste à appliquer de manière répétée une composition contenant un agent oxydant puissant acceptable pour les usages cosmétiques sur le cuir chevelu chauve de manière à porter la composition en contact avec les glandes sébacées du cuir chevelu.

2. Un procédé selon la revendication 1, dans lequel l'agent oxydant consiste en dioxyde de chlore stabilisé, bromate de sodium, bromate de potassium ou perborate de sodium.

3. Un procédé selon la revendication 2, dans lequel le dioxyde de chlore stabilisé est présent dans la composition en quantités de 0,01 à 0,05% (100 à 500 ppm).

4. Un procédé selon la revendication 2, dans lequel le bromate de sodium est présent dans la composition en quantités de 5 à 12% (50 000 à 120 000 ppm).

5. Un procédé selon la revendication 2, dans lequel le bromate de potassium est présent dans la composition en quantités de 5 à 12%.

6. Un procédé selon la revendication 2, dans lequel le perborate de sodium est présent dans la composition en quantités d'environ 5%.

FIG.1.

FIG.2.